# EUROPEAN PATENT SPECIFICATION

(11) **EP 4 744 603 B1**
(45) Date of publication and mention of the grant of the patent: **11.11.2026**
(21) Application number: 24213714.9
(22) Date of filing: 18.11.2024
(51) Int. Cl.: A61B 17/70, A61B 90/00

(54) **COUPLING DEVICE FOR COUPLING A ROD TO A BONE ANCHORING ELEMENT**
KUPPLUNGSVORRICHTUNG ZUR VERBINDUNG EINER STANGE MIT EINEM KNOCHENVERANKERUNGSELEMENT
DISPOSITIF DE COUPLAGE POUR COUPLER UNE TIGE À UN ÉLÉMENT D'ANCRAGE OSSEUX

(43) Date of publication of application: 20.05.2026
(73) Proprietor: Biedermann Technologies GmbH & Co. KG, 78166 Donaueschingen (DE)
(72) Inventor: BIEDERMANN, Timo, 78647 Trossingen (DE); SCHÜNEMANN, Achim, 78054 Villingen-Schwenningen (DE); FISCHER, Bernd, 79877 Friedenweiler (DE)
(74) Representative: Prüfer & Partner mbB Patentanwälte · Rechtsanwälte

(56) References cited:
- US-A1- 2021 401 466
- US-A1- 2023 093 687

## Description

The invention relates to a coupling device for coupling a rod to a bone anchoring element. In particular, the invention relates to a coupling device that forms part of a polyaxial bone anchoring device.

In spinal surgery, often multiple segments of the spinal column have to be corrected and/or stabilized using a spinal rod and polyaxial bone anchors. During such a procedure, repeated adjustments of the bone anchoring elements and the rod relative to the receiving parts of a respective polyaxial bone anchoring devices may become necessary.

Usually, a polyaxial bone anchoring device includes a coupling device and a bone anchoring element with a head that is pivotably received in the coupling device and can be locked at a desired angle of the bone anchoring element relative to the coupling device. The coupling device also receives a rod that is configured to connect the polyaxial bone anchoring device to a further bone anchor.

US 2021/0401466 A1 discloses a polyaxial bone anchoring device including a receiving part with an upper member and a lower member connectable to the upper member, and a pressure member positionable in the upper member to exert pressure on a head of a bone anchoring element.

US 2023/0093687 A1 discloses a bone anchoring device including a receiving part having a rod receiving portion and a head receiving portion, and a locking ring configured to be arranged around the head receiving portion for locking an inserted head.

US 10, 258,383 B2 describes a polyaxial bone anchoring device comprising a receiving part for coupling a rod to a bone anchoring element, the receiving part comprising a rod receiving portion, a head receiving portion for accommodating a head of the bone anchoring element, and a locking ring configured to be arranged around the head receiving portion. The locking ring can assume a locking position where the locking ring exerts a force on the head receiving portion of the receiving part to lock an inserted head and wherein the locking ring can be moved relative to the receiving part from the locking position towards a position where the inserted head is pivotable. With such a polyaxial bone anchoring device and instrument, a temporary locking and unlocking of the head in the receiving part can be carried out several times without using a separate locking element.

US 11,213,323 B1 describes a coupling device including a receiving part having a head receiving portion for pivotably receiving a head of a bone anchor and a rod receiving portion defining a recess for receiving the rod, the rod receiving portion having a first engagement surface for engaging an instrument. The coupling device further includes a locking ring positionable around the head receiving portion and having a second engagement surface for engaging the instrument. With the instrument, the locking ring can be moved into a pre-locking position after placement of the coupling device onto the head of the bone anchor. This prevents removal of the coupling device from the head of an inserted bone anchor once the coupling device has been connected to the head of the bone anchor. The coupling device and the instrument are applicable for *in-situ* placement of the coupling device on a bone anchor already inserted into bone.

In particular during *in-situ* placement of the coupling device and during manipulating steps carried out during surgery, it is essential that the head of a bone anchoring element is safely clamped and that at the same time the force necessary for mounting the coupling device on the head is moderate.

It is the object of the invention to provide a coupling device for coupling a bone anchoring element to a rod, in particular in a polyaxial manner, and a polyaxial bone anchoring device with such a coupling device which has an increased strength and stability.

The object is solved by a coupling device according to claim 1 and by a polyaxial bone anchoring device according to claim 16. Further developments are given in the dependent claims.

A coupling device for coupling a rod to a bone anchoring element having a shank and a head according to an embodiment includes a receiving part having a first end, a second end, a central axis extending between the first end and the second end and a passage extending from the first end to the second end; the receiving part further including a rod receiving portion having a recess defining a channel for receiving the rod, the recess extending from the first end towards the second end and forming two free legs and having a bottom; and a head receiving portion open to the second end and comprising a seat portion for pivotably receiving the head of the bone anchoring element, the head receiving portion being flexible for inserting and clamping the head; and a locking ring configured to be arranged around the head receiving portion, wherein, when the locking ring is around the head receiving portion, the locking ring can assume a locking position where the locking ring exerts a force on the head receiving portion to lock an inserted head; and wherein the passage comprises a widening section that widens from the seat portion towards the bottom of the recess.

Due to the widening section, the coupling device has an increased strength or stiffness without requiring an increased force necessary for mounting the coupling device on the head of the bone anchoring element. Thus, an in-situ placement of the coupling device onto a head of an inserted bone anchoring element can be achieved gently without exerting excessive forces that may cause injuries.

In addition, the clamping force that the coupling device exerts on the head of the bone anchoring element may be increased. Also, inadvertent detaching of the locking ring from the receiving part may be avoided.

The coupling device permits, once placed onto the head of the bone anchor, a temporary locking without the requirement of a rod or an additional locking member. The rod can even be inserted and is not required to sit on the rod support but can be at an elevated position. Hence, by moving the locking ring from a locking position to a pre-locking position and vice versa, the bone anchoring device can be locked and unlocked to permit various adjustment steps. As such, the rod and/or the locking member are only truly needed for the final locking at the end of the surgical procedure. Therefore, the *in-situ* placement of the coupling device with the capability of temporary and/or variable locking of the head increases the variety of correction steps that can be carried out during surgery.

A polyaxial bone anchoring device according to embodiments comprises in addition to the coupling device a bone anchoring element having a head and a shank, wherein the head has a spherically-shaped outer surface portion.

The bone anchoring element may also be assembled with the coupling device prior to insertion into bone. Hence, the surgeon has a wide variety of handling the polyaxial bone anchoring device.

According to another embodiment a coupling device for coupling a rod to a bone anchoring element having a shank and a head according to an embodiment includes a receiving part having a first end, a second end, a central axis extending between the first end and the second end and a passage extending from the first end to the second end; the receiving part further including a rod receiving portion having a recess defining a channel for receiving the rod, the recess extending from the first end (towards the second end and forming two free legs and having a bottom; and a head receiving portion open to the second end and comprising a seat portion for pivotably receiving the head of the bone anchoring element, the head receiving portion being flexible for inserting and clamping the head; and a locking ring configured to be arranged around the head receiving portion, wherein, when the locking ring is around the head receiving portion, the locking ring can assume a locking position where the locking ring exerts a force on the head receiving portion to lock an inserted head; and wherein the head receiving portion comprises adjacent to the second end an inner conical surface portion that narrows towards the seat portion and that has an axial length corresponding to the axial heigth of the portion of an inserted head that projects out of the seat portion up to the shank when the shank of the bone anchoring element is coaxial with the central axis.

Further features and advantages of the invention will become apparent from the description of embodiments by means of the accompanying drawings. In the drawings:
- Fig. 1:: shows a perspective exploded view of a polyaxial bone anchoring device with a coupling device and a rod according to a first embodiment.
- Fig. 2:: shows a perspective view of the polyaxial bone anchoring device of Fig. 1 in the assembled state.
- Fig. 3:: shows a cross-sectional view of the polyaxial bone anchoring device of Fig. 2, the cross-section taken in a plane perpendicular to the rod axis of the inserted rod.
- Fig. 4:: shows a perspective view from a top of the receiving part of the coupling device according to Figs. 1 to 3.
- Fig. 5:: shows a perspective view from a bottom of the receiving part of Fig. 4.
- Fig. 6:: shows a top view of the receiving part of Figs. 4 and 5.
- Fig. 7:: shows a cross-sectional view of the receiving part of Figs. 4 to 6, the cross-section taken along line A-A in Fig. 6.
- Fig. 8:: shows a perspective view from a top of a locking ring of the coupling device according to Figs. 1 to 3.
- Fig. 9:: shows a perspective view from a bottom of the locking ring of Fig. 8.
- Fig. 10:: shows a top view of the locking ring of Figs. 8 and 9.
- Fig. 11:: shows a cross-sectional view of the locking ring of Figs. 8 to 10, the cross-section taken along line B-B in Fig. 10.
- Figs. 12 to 15:: show cross-sectional views of steps of assembling the polyaxial bone anchoring device of Figs. 1 to 3 and locking of the head, wherein the cross-section is taken in a plane perpendicular to the rod axis.

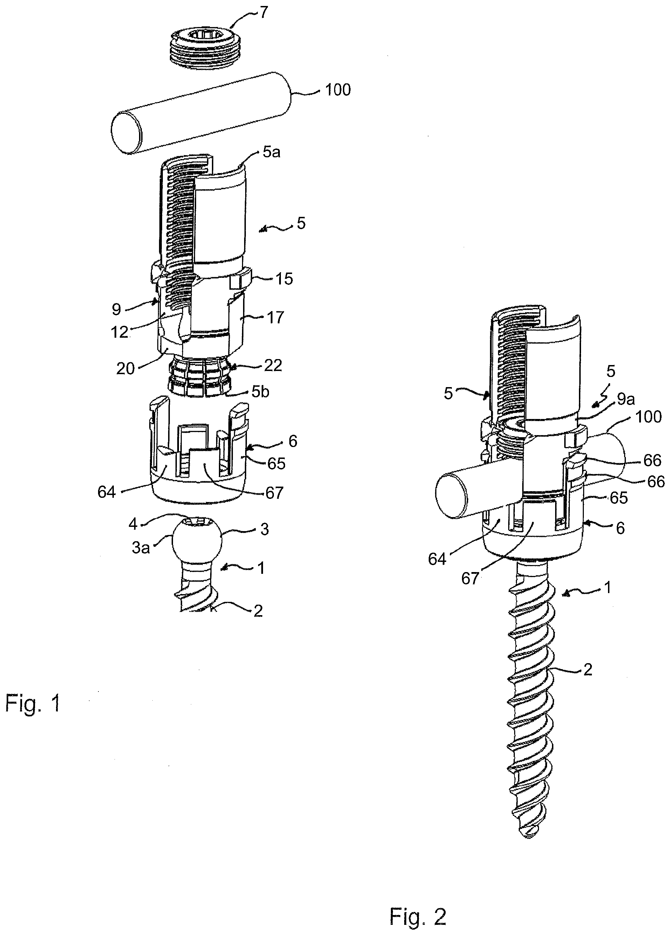

As shown in Figs. 1 to 3, a polyaxial bone anchoring device according to an embodiment includes a bone anchoring element 1 in the form of, for example, a bone screw having a shank 2 with a threaded portion and a head 3 with a spherically-shaped outer surface portion 3a. The head 3 has a recess 4 for engagement with a driver or tool. The polyaxial bone anchoring device also includes a coupling device comprising a receiving part 5 for receiving a rod 100 to be connected to the bone anchoring element 1 and a locking ring 6. The locking ring 6 is configured to extend around a portion of the receiving part 5 for clamping and locking the head 3 in the receiving part 5. Further, a fixation element 7 in the form of, for example, an inner screw or set screw, may be provided for fixing the rod 100 in the receiving part 5. The polyaxial bone anchoring device is configured to permit coupling of the rod to the bone anchoring element in various angular positions of the coupling device relative to the shank 2 of the bone anchoring element 1.

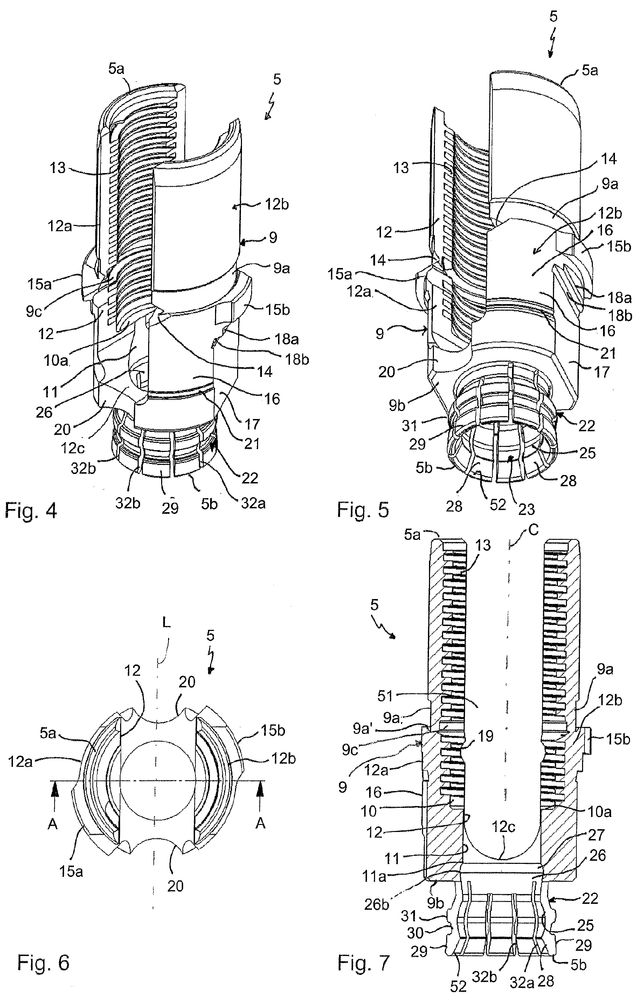

Referring additionally to Figs. 4 to 7, the receiving part 5 may preferably be a monolithic part, and has a first end or top end 5a, a second end or bottom end 5b and a passage 51 extending from the top end 5a towards the bottom end 5b, the passage 51 defining a longitudinal central axis C. By the passage 51 an opening 52 at the bottom end 5b is defined, which has a width that is greater than a greatest width of the head 3, so that the head 3 of the bone anchoring element 1 is insertable through the opening 52. The receiving part comprises a rod receiving portion 9 and a head receiving portion 22. Adjacent to the top end 5a, the receiving part 5 comprises the rod receiving portion 9, which may have an outer shape that is substantially cylindrical. At a distance from the top end 5a, the rod receiving portion comprises a bottom end 9b. A coaxial first bore 10 is provided in the rod receiving portion 9 that extends from the top end 5a towards a first distance from the bottom end 9b of the rod receiving portion. The rod receiving portion 9 further includes a coaxial second bore 11 extending from a lower end of the first bore 10 to a second distance from the bottom end 9b of the rod receiving portion. A diameter of the second bore 11 may be smaller than the diameter of the first bore 10 and smaller than a diameter of the head 3 of the bone anchoring element 1.

A substantially U-shaped recess 12 extends from the top end 5a of the receiving part in the direction of the bottom end 9b in the rod receiving portion 9, wherein a width of the recess 12 is slightly larger than a diameter of the rod 100, such that the rod 100 can be placed in the recess 12 and can be guided therein. The recess 12 forms a channel for the rod 100. By means of the recess 12, two free legs 12a, 12b are formed, on which an internal thread 13 may be provided. The internal thread 13 can be, for example, a metric thread, a flat thread, a negative angle-thread, a saw-tooth thread, or any other thread form. Preferably, a thread form such as a flat thread or a negative angle thread is used to prevent or reduce splaying of the legs 12a, 12b when the fixation element 7 is screwed-in. It shall be noted, that the internal thread 13 extends to a small distance from a bottom 10a of the bore 10 and there is no undercut provided between the lower end of the internal thread 13 and the bottom 10a of the bore 10. Hence, the section of the inside of the rod receiving portion 9 below the internal thread 13 is free from an undercut. This results in an enhanced wall strength in the lower region of the legs 12a, 12b which renders them stiffer.

At a distance from the top end 5a, an external groove 9a or otherwise weakened section is provided that has a reduced wall thickness and allows breaking-off of the upper portion of the legs 12a, 12b. The external groove 9a may have a lower wall 9a' which extends at an acute angle with respect to the central axis C as best seen in Fig. 7. At an edge of the external groove 9a in a circumferential direction, recesses or cutouts 14 may be formed at either side of the rod channel and at both ends in the longitudinal direction of the channel. The cutouts 14 may facilitate breaking-off of the upper portion of the legs 12a, 12b. Inside the bore 10, the internal thread 13 may be interrupted by means of an internal groove 9c at an axial position that substantially corresponds to a lower edge of the external groove 9a. Hence, the leg portions above the external groove 9a can serve as extended tabs. With the extended tabs, it is possible to manipulate the polyaxial bone anchoring device with an inserted rod that may be at a higher position with respect to a bottom 12c of the recess 12. By means of this, for example, a vertebra can be pulled against the rod.

The depth of the recess 12 may be such that when the rod 6 is placed into the recess 12 and the fixation element 7 is screwed between the legs 12a, 12b, the locking element 7 does not substantially protrude out of the receiving part 5 when the extended tabs have been broken-off.

At an outer surface of the rod receiving portion 9, an engagement structure for engagement with an instrument (not shown) may be provided. The engagement structure may include circumferentially extending ribs, which may be arranged asymmetrical with respect to a plane including the central axis C of the receiving part 5 and a channel axis L of the substantially U-shaped recess 12. That means, a first rib 15a can start at a distance from the edge of the U-shaped recess 12 on one side and extend to a distance around the rod receiving portion 9. A second rib 15b can start at the opposite side relative to the U-shaped recess and extend to a distance around the rod receiving portion 9. More specifically, the position of the ribs 15a, 15b is offset by 180° measured in relation to the central axis C, and is rotated with respect to the central axis C such that the ribs 15a of the leg 12a extend to the rod receiving recess 12 at one side of the receiving part 5 and the ribs 15b extend to the rod receiving recess 12 at the other side of the receiving part 5. Thereby, a rib-free surface 16 is formed on each side of the U-shaped recess 12. This permits the instrument to be placed first onto the rib-free surface, and then to be rotated to engage the ribs 15a, 15b. The ribs 15a, 15b may have a substantially rectangular cross-section and may have inclined end portions. It shall be understood that more than one rib can be provided in the axial direction. Also, the shape of the ribs may be different in other embodiments. Below each of the ribs 15a, 15b, a substantially flat outer surface portion 17 may be formed that may have a contour of a rectangle or square and may serve for engagement with a portion of the locking ring 6. Between the ribs 15a, 15b and the substantially flat surface portion 17, two latching grooves 18a, 18b may be provided one after the other in an axial direction. The latching grooves 18a, 18b are configured to cooperate with a portion of the locking ring 6 to provisionally hold the locking ring 6 at one or more specific positions. The upper latching groove 18a may serve for preventing further upward movement of the locking ring in an insertion position. The lower latching groove 18b may serve for latching the locking ring in a pre-locking position.

At an axial position sligthly below the internal groove 9c and above the bottom 10a of the bore 10, an engagement structure 19, for example in the form of a shallow groove, for engagement with the instrument may be provided on the inner surface of the bore 10.

Furthermore, cutouts 20 may be provided on either side of the substantially U-shaped recess 12, which may serve for receiving projections of the locking ring 6 therein. By means of this, the locking ring 6 can be secured against rotation relative to the receiving part 5. The cutouts 20 have a rounded, preferably a cylinder segment-shaped contour. Compared to a rectangular or square-shaped cutout, the rounded cut-out 20 results in an enhanced stiffness of the receiving part 5, in particular of the rod receiving portion 9.

Moreover, a circumferentially extending groove 21 may be formed on the outer surface of the rod receiving portion 9 at an axial position above the cutouts 20 and below the ribs 15a, 15b. The groove 21 may serve as a further abutment for the locking ring 6.

The head receiving portion 22 extends from the bottom end 9b of the rod receiving portion to the bottom end 5b of the receiving part 5. It has a substantially cap-like shape with a hollow interior portion 23 providing a seat for receiving the head 3 pivotably therein. A greatest outer diameter of the head receiving portion 22 is smaller than a greatest outer diameter of the rod receiving portion 9. A free end of the head receiving portion 22 forms the bottom end 5b of the receiving part 5 with the passage 51 ending in the opening 52. In greater detail, the passage 51 comprises in the head receiving portion 22 a seat portion 25 for the head, which has preferably a spherical shape adapted to the shape of the spherically-shaped portion 3a of the head 3. Moreover, the seat portion 25 is configured to encompass the head 3 of the bone anchoring element 1 from the side, to cover a region including the largest diameter of the head 3.

Between the seat portion 25 and the second bore 11 of the rod receiving portion a widening section 26 that widens towards the rod receiving portion 9, preferably a conically widening section, is formed. The widening section 26 extends into the rod receiving portion 9. Adjacent to the seat portion 25, the widening section 26 has an inner diameter substantially matching an inner diameter of the seat portion 25 at this position. At its upper end, the widening section 26 has an inner diameter that is smaller than an inner diameter of the first bore 10 and greater than an inner diameter of the second bore 11. Between the lower end of the second bore 11 and the widening section 26, a small transition section 27 is formed that narrows towards the second bore 11. The transition section 27 ends a small distance from the bottom 12c of the U-shaped recess 12. The size of the widening section is designed such that the strength of the receiving part 5 is increased. In particular, the stiffness of the legs 12a, 12b may be increased.

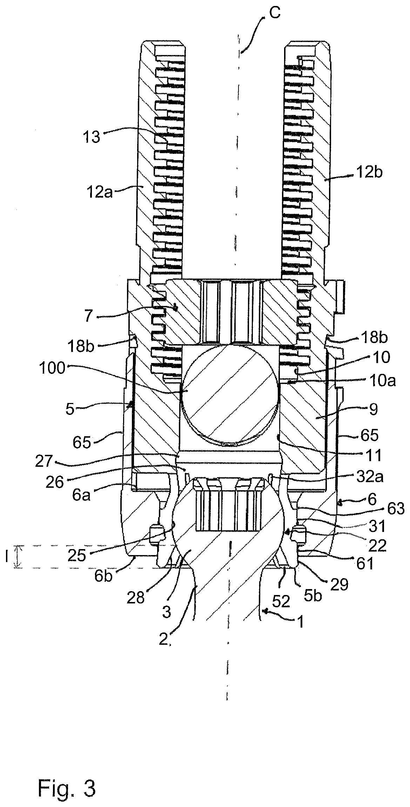

Adjacent to the bottom end 5b of the receiving part 5, the passage 51 comprises a widening section 28, preferably a conically widening section, that widens towards the bottom end 5b of the receiving part 5. The widening section 28 forms an angle with the central axis C. This angle defines the maximum pivot angle that the shank 2 can form with the central axis C. For example, the angle may be 30° or more. As best seen in Fig. 3, the axial length 1 of the widening section 28 may correspond substantially to the axial length of the portion of the head 3 that extends out of the seat portion 25 up to the shank 2, when the head 3 is received in the seat portion 25 and the shank 2 is coaxial with the central axis C. With such an axial length 1 of the widening section 28, the head receiving portion 22 projects out of the locking ring 6 to such an extent that inadvertent moving of the locking ring 6 past the bottom end 5b of the receiving part 5 may be avoided.

An outer surface of the head receiving portion 22 may be recessed in a radial direction relative to the bottom end 9b of the rod receiving portion 9. The outer surface of the head receiving portion 22 has a circumferentially extending lowermost outer section 29 that is conically widening in the direction of the bottom end 5b of the receiving part 5. Preferably, the angle of the lowermost outer section 29 is about 4° to 6 °, preferably about 5°, more preferably 5°, relative to the central axis C. The lowermost outer section 29 is configured to cooperate with a corresponding portion of the locking ring 6 to exert a compression force onto an inserted head. The axial length of the lowermost outer section 29 may correspond to the axial length of the widening section 28 of the hollow interior 23. Following the lowermost outer section 29 a circumferential groove 30 is formed which is followed by a circumferentially extending and radially outwardly bulged section 31. The radially outwardly bulged section 31 is configured to cooperate with another portion of the locking ring 6 to further exert a clamping force onto an inserted head 3. The bulged section 31 narrows in the direction towards the rod receiving portion 9, which contributes to the recessed shape of the head receiving portion 22.

A plurality of slits 32a, 32b render the head receiving portion 22 flexible, so that, when the head 3 is inserted into the hollow interior 23, the head receiving portion 22 expands. When pressure is exerted onto an inserted head 3 by the locking ring 6, the head receiving portion 22 is compressed. By means of this, the head 3 can be clamped or locked depending on the friction force between the head 3 and the sat portion 25. The slits 32a, 32b are open towards the bottom end 5b of the receiving part 5. A first type of slits 32a extends into the widening section 26 up to about an axial position of the bottom end 9b of the rod receiving portion 9. A second type of slits 32b,which is shorter than the first type of slits 32a, extends only slightly above the upper end of the seat portion 25. The second type of slits 32b may be arranged circumferentially at both sides of the center of the U-shaped recess 12. In the embodiment, four shorter slits 32b are provided, two on either side of the U-shaped recess 12. The first type of slits 32a may be arranged at the position of the legs 12a, 12b, respectively, as can be seen in Figs. 4, 5 and 7. The shorter slits may enhance the strength of the head receiving section 22 and therewith the clamping force. The number of slits may vary according to the desired flexibility of the head receiving portion 22..

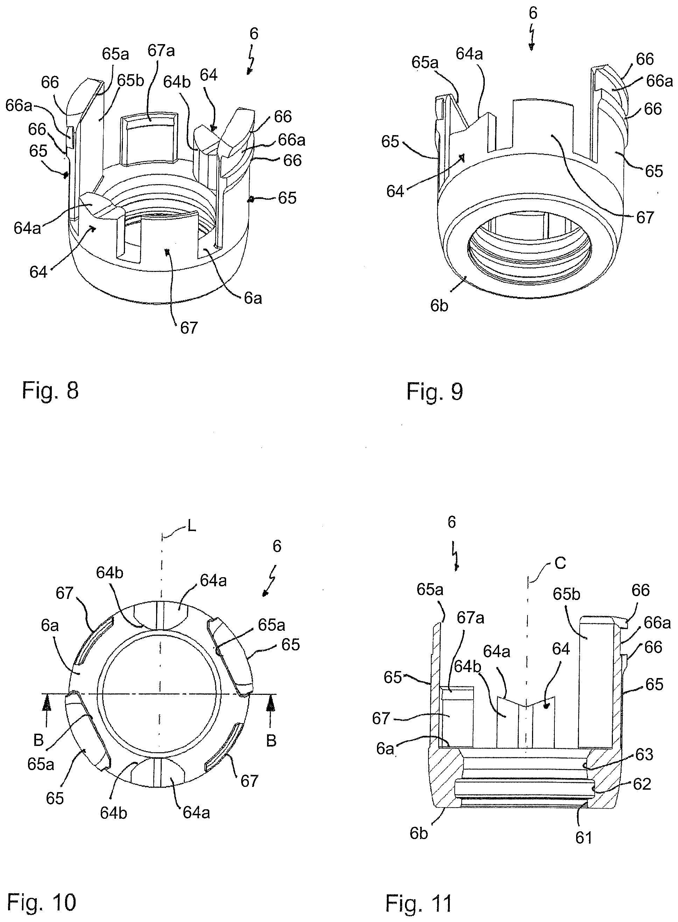

Turning now to Figs. 8 to 11, the locking ring 6 will be described. The locking ring 6 is designed to encompass the head receiving portion 22 and has an internal surface structure that facilitates, in cooperation with the head receiving portion 22, a full locking of an inserted head 3 in the head receiving portion 22 when the locking ring is at its lowermost position. It further enables a pre-locking when the locking ring 6 is at a position slightly above the locking position, which still allows pivoting of the head 3 in the head receiving portion 22, but prevents removal of the head 3 from the head receiving portion 22. Lastly, the locking ring 6 is configured to permit insertion of the head 3 into the hollow interior 23 when the locking ring is at an insertion position which is defined by an uppermost position the locking ring can assume relative to the receiving part 5.

In greater detail, the locking ring 6 has a lower surface 6b and an opposite upper surface 6a that is substantially ring-shaped. Adjacent to the lower surface 6b, there is a first annular projection 61 which projects inwardly and is configured to cooperate with the lowermost outer section 29 of the head receiving portion 22. The annular projection 61 may have a cylindrical inner surface or a slightly tapered inner surface. Following the annular projection 61, there is a widened section 62, which is then followed towards the upper surface 6a by a bulged section 63 that also projects inwardly. As depicted in Fig. 3, when the locking ring 6 is mounted around the head receiving portion 22, the first annular projection 61 is configured to press onto the lowermost outer section 29 of the head receiving portion 22 and the bulged section 63 is configured to press onto the bulged section 31 of the head receiving portion 22.

From the upper surface 6a of the ring-shaped portion of the locking ring 6, two rod support projections 64 protrude upwardly and have a free end surface forming a rod support surface 64a for the rod 100. An inner surface 64b of the projections 64 is rounded so as to mate with the shape of the cutouts 20, preferably, the inner surface 64b is cylindrical. The rod support projections 64 are diametrically opposite, i.e., offset by 180°, from one another. A cross-section of the rod support surface 64a may be substantially V-shaped to permit safe support of rods of different diameters. When the locking ring 6 is mounted on the receiving part 5 such that the upper surface 6a faces towards the top end 5a of the receiving part, the rod support projections 64 may extend through the cutouts 20 in the rod receiving portion 9 and thereby secure the rotational orientation of the locking ring 7 relative to the receiving part 5.

The locking ring 6 also includes two upstanding arms 65 that are positioned asymmetrically with respect to a plane that extends through the central axis C and through the centers of the rod support surfaces 64a, for example, in the same or similar manner as the ribs 15a, 15b of the rod receiving portion 9 are arranged on the receiving part 5. At an upper end of the arms 65, an engagement portion in the form of circumferential ribs 66 defining a groove 66a are provided for engagement with an instrument. An inwardly facing upper edge 65a of the arms 65 may be provided that is configured to engage the latching grooves 18a, 18b at the receiving part 5. In the assembled state, the engagement structure in the form of the ribs 66 with the groove 66a is aligned with the engagement structure 15a, 15b at the receiving part 5, leaving the rib-free surface 16 of the receiving part 5 exposed. Moreover, the arms 65 have a substantially flat inner wall 65b that is configured to engage the flat portion 17 at the rod receiving portion 9. Thereby, a further form-fit connection is established between the locking ring 7 and the receiving part 5. Between the rod support projections 64 and the arms 65, there are upstanding wall portions 67 that have a height slightly greater than that of the rod support projections 64 and which have at an inner wall thereof a ledge 67a may engage the groove 21 at head receiving portion 9 of the receiving part 5.

A bone anchoring device according to an embodiment may further include the rod 100 that is configured to be inserted into the substantially U-shaped recess 12 and the fixation element 7, such as a set screw, that is configured to be screwed between the legs 12a, 12b. Thus, the fixation element 7 comprises a thread corresponding to the internal thread 13 provided on the legs 12a, 12b.

The coupling device and parts thereof as well as the bone anchoring element and the locking element and the rod may be made of any bio-compatible material, preferably, however, of titanium or stainless steel or of any other bio-compatible metal or metal alloy or plastic material. As a bio-compatible alloy, a NiTi alloy, for example Nitinol, may be used. Other materials can also be magnesium or magnesium alloys. Bio-compatible plastic materials for use may be, for example, polyether ether ketone (PEEK) or poly-L-lactide acid (PLLA). The coupling device and other parts of the polyaxial bone anchoring device may be made of the same or of a different material.

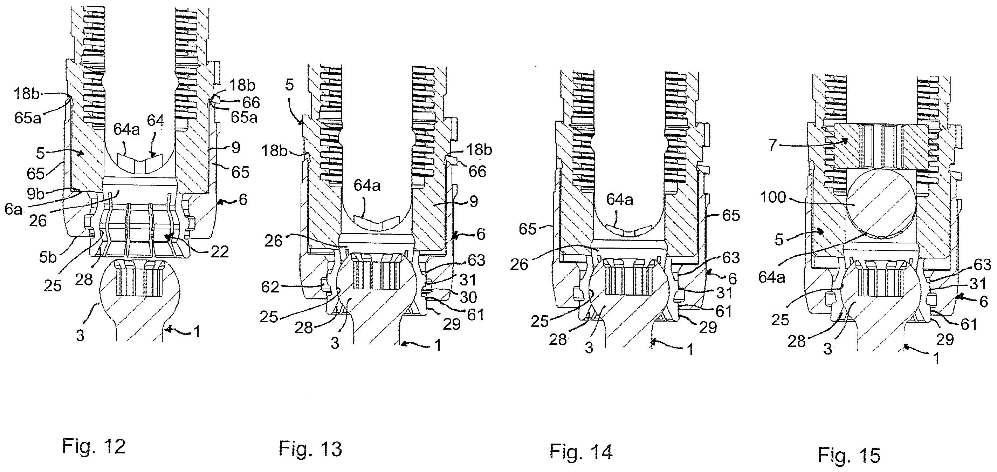

Steps of assembling the coupling device with the bone anchoring element 1 will be described with reference to Figs. 12 to 15.

The coupling device may include the receiving part 5 and the locking ring 6 in a pre-assembled manner. It shall be noted that the locking ring 6 can be mounted on the receiving part 5 from the bottom end 5b by compressing the head receiving portion 22. When mounted, the rod support projections 64 extend into the cutouts 20 of the receiving part. The bone anchoring element 1 may be placed already into bone, so that the coupling device can be assembled with the bone anchoring element *in-situ.* Alternatively, the coupling device can be assembled with the bone anchoring element 1 prior to insertion into bone.

As shown in Fig. 12, the locking ring 6 is in the insertion position in which the annular projection 61 of the locking ring 6 is approximately at the axial height of the groove 30 in the outer surface of the head receiving portion 22. The upper surface the arms 65 of the locking ring 6 may engage the upper groove 18a at the outer surface of the rod receiving portion 9. In the insertion position, the head 3 can be inserted into the hollow interior 23 of the head receiving portion 22. Due to the flexibility of the head receiving portion 22, the head receiving portion snaps over the head 3 such that the head 3 it is seated in the seat portion 25 may be held there by friction.

As shown in Fig. 13, the locking ring 6 is moved slightly downward towards the bottom end 5b of the receiving part 5 until the arms 65 snap into the lower groove 18b at the rod receiving portion 9. The locking ring 6 is now latched at a pre-locking position. In the pre-locking position, the annular projection 61 of the locking ring 6 presses on an upper portion of the lowermost outer section 29 of the head receiving portion 22 and the bulge section 63 of the locking ring 6 slightly presses onto the outwardly bulged section 31 of the head receiving portion 22. The head receiving portion 22 is compressed to such an extent, that the head 3 cannot escape through the lower opening 52. The head may be held by friction in the seat portion 25 so that the coupling device can assume an angular position relative to the shank which can be maintained provisionally prior to locking.

Next, as depicted in Fig. 14, the locking ring 6 is moved further downward. Due to the increasing compression force acting onto the head receiving portion 22 when the annular projection of the locking ring slides along the widening lowermost outer surface portion 29 of the head receiving portion 22, the head 3 is clamped in the head receiving portion 22. The clamping force can have such a strength, that the head may be locked temporarily therein.

In the configuration shown in Fig. 14 when moving the locking ring 6 with an instrument upwards out of the temporary locking position, the head can be unlocked from temporary locking and may be pivoted again. Locking and unlocking can be carried out with the instrument several times. Neither a rod nor a fixation member needs to be present in the rod channel for locking and unlocking of the head.

Finally, when a suitable angular position of the polyaxial bone anchoring device is found, the rod 100 may be inserted and the fixation member 7 may be placed between the legs 12a, 12b and tightened to finally lock the head as shown in Fig. 15. Finally, the upper portion of the legs 12a, 12b which served as extended tabs are broken-off.

In clinical use, a plurality of polyaxial bone anchoring devices is inserted into bone parts or into vertebrae, in particular into the pedicles of vertebrae. The coupling devices are then aligned so that a rod (not shown) can be received in the rod receiving portions of two or more of the bone anchoring devices.

Modifications of the above described embodiments are conceivable. In particular, the shape of the parts is not limited to the detailed shape shown in the figures. Deviations may be possible and encompassed by the disclosure.

Instead of the locking member being a set screw all other kinds of locking assemblies known in the art may be used. For the bone anchoring element, all types of bone anchoring elements are suitable for anchoring in bone or a vertebra, such as bone screws, bone nails, etc. may be used. The rod can be any elongate device that is configured to connect two bone anchoring devices. The rod may have various shapes and/or varying cross-section along its length. The rod may be stiff or more flexible.

The spherical outer surface portion of the head may be only partly spherical seen in the circumferential direction and the seat portion in the head receiving portion may be adapted thereto such that pivoting of the head is possible only in a predefined plane.

Moreover, the head receiving portion may have a design that allows to pivot the bone anchoring element to a greater pivot angle to one side compared to other sides.

## Claims

1. A coupling device for coupling a rod (100) to a bone anchoring element (1) having a head (3) and a shank (2),
the coupling device including
a receiving part (5) having a first end (5a), a second end (5b), a central axis (C) extending between the first end (5a) and the second end (5b) and a passage (51) extending from the first end (5a) to the second end (5b); the receiving part (5) further including
a rod receiving portion (9) having a recess (12) defining a channel for receiving the rod (100), the recess extending from the first end (5a) towards the second end (5b) and forming two free legs (12a, 12b) and having a bottom (12c); and
a head receiving portion (22) open to the second end (5b) and comprising a seat portion (25) for pivotably receiving the head (3) of the bone anchoring element (1), the head receiving portion being flexible for inserting and clamping the head (3); and
a locking ring (6) configured to be arranged around the head receiving portion (22),
wherein, when the locking ring (6) is around the head receiving portion (22), the locking ring (6) can assume a locking position where the locking ring (6) exerts a force on the head receiving portion (22) to lock an inserted head (3); and
wherein the passage (51) comprises a widening section (26) that widens from the seat portion (25) towards the bottom (12c) of the recess (12).

2. The coupling device of claim 1, wherein the widening section (26) has a conical shape.

3. The coupling device of claim 1 or 2, wherein the passage (51) comprises a coaxial bore (11) in an axial region extending along the bottom (12c) of the recess (12) and wherein the widening section (26) comprises a lower end facing towards the seat portion (25) and an upper end facing towards the bottom (12c) of the recess (12) and wherein an inner width at the upper end of the widening section (26) is greater than a diameter of the bore (11).

4. The coupling device of claim 3, wherein the passage (51) comprises between the upper end of the widening section (26) and the bottom (12c) of the recess (12) a transition section (27) tapering towards the bore (11).

5. The coupling device of one of claims 1 to 4, wherein the head receiving portion (22) comprises adjacent to the second end (5b) an inner conical surface portion (28) that narrows towards the seat portion (25) and that has an axial length (l) corresponding to a portion of an inserted head (3) that extends out of seat portion (25) when the shank (2) of the bone anchoring element is coaxial with the central axis (C).

6. The coupling device of claim 5, wherein the inner conical surface portion (28) forms an angle with the central axis (C) of 30° or more.

7. The coupling device of one of claims 1 to 6, wherein the head receiving portion comprises adjacent to the second end (5b) an outer conical surface portion (29) that widens towards the second end (5b), preferably wherein the outer conical surface portion forms an angle with the central axis (C) that is between about 4° and 6°, more preferably about 5°.

8. The coupling device of one of claims 1 to 7, wherein the head receiving portion (22) comprises a plurality of axially extending slits (32a) of a first type that are open towards the second end and wherein at least one of the slits (32a) has a closed end opposite to the second end (5b) that is located at an axial position within the widening section (26), preferably wherein at least one, more preferably two or more of the slits (32a) of the first type are located at a circumferential position outside the position of the recess (12).

9. The coupling device of one of claims 1 to 8, wherein the head receiving portion (22) comprises a plurality of axially extending slits (32b) of a second type that are open towards the second end (5b) and wherein at least one of the slits (32b) has a closed end opposite to the second end (5b) that is located at an axial position closer to the seat portion (25) than to the bottom (12c) of the recess (12), preferably wherein at least one, more preferably two or more of the slits (32b) of the second type, are located at a circumferential position corresponding to the position of the recess (12).

10. The coupling device of claim 8 or 9, wherein the slits (32a) of the first type are axially longer than the slits (32b) of the second type.

11. The coupling device of one of claims 1 to 10, wherein the legs (12a, 12b) comprise extended tabs that can be broken-off.

12. The coupling device of one of claims 1 to 11, wherein the passage (51) comprises a first bore (10) adjacent to the first end (5a), the first bore comprising an internal thread (13) and wherein the first bore (10) is free from an undercut at a lower end of the internal thread (13).

13. The coupling device of one of claims 1 to 12, wherein the rod receiving portion (9) comprises at a position below the bottom (12c) of the recess (12) cutouts (20) at either end of the channel for receiving projections (64) of the locking ring (6) therein and wherein a contour of the cutouts (20) corresponds to a contour of the projections (64), wherein the contour is rounded, preferably cylindrical.

14. The coupling device of one of claims 1 to 13, wherein the rod receiving portion (9) comprises a substantially cylindrical outer surface with a first outer width and wherein the head receiving portion (22) comprises adjacent to the rod receiving portion (9) a second outer width that is smaller than the first outer width such that a step is formed between a bottom end (9b) of the rod receiving portion (9) and the head receiving portion (22).

15. The coupling device of one of claims 1 to 14, wherein the rod receiving portion (9) further has an outer surface and a first engagement structure (15a, 15b) on the outer surface and the locking ring (6) has an outer surface and a second engagement structure (66, 66a) on the outer surface and wherein the first engagement structure (15a, 15b) and the second engagement structure (66, 66a) are configured to be engaged by an instrument to move the locking ring (6) relative to the receiving part (5) along the central axis (C) from the locking position towards the first end (5a) to a position where the inserted head (3) is pivotable.

16. A polyaxial bone anchoring device including a coupling device according to any one of claims 1 to 15 and a bone anchoring element (1) having a shank (2) and a head (3) wherein the head (3) comprises a spherical outer surface portion (3a).

## Patentansprüche

1. Kopplungsvorrichtung zum Koppeln eines Stabs (100) an ein Knochenverankerungselement (1) mit einem Kopf (3) und einem Schaft (2),
wobei die Kopplungsvorrichtung umfasst
ein Aufnahmeteil (5) mit einem ersten Ende (5a), einem zweiten Ende (5b), einer Mittelachse (C), die sich zwischen dem ersten Ende (5a) und dem zweiten Ende (5b) erstreckt, und einem Durchgang (51), der sich von dem ersten Ende (5a) zu dem zweiten Ende (5b) erstreckt; wobei das Aufnahmeteil (5) weiter umfasst
einen Stabaufnahmeabschnitt (9) mit einer Ausnehmung (12), die einen Kanal zum Aufnehmen des Stabs (100) definiert, wobei sich die Ausnehmung von dem ersten Ende (5a) in Richtung des zweiten Endes (5b) erstreckt und zwei freie Schenkel (12a, 12b) bildet und einen Boden (12c) aufweist; und
einen Kopfaufnahmeabschnitt (22), der zu dem zweiten Ende (5b) offen ist und einen Sitzabschnitt (25) zum schwenkbaren Aufnehmen des Kopfs (3) des Knochenverankerungselements (1) umfasst, wobei der Kopfaufnahmeabschnitt zum Einführen und Klemmen des Kopfs (3) flexibel ist; und
einen Verriegelungsring (6), der dazu ausgebildet ist, um den Kopfaufnahmeabschnitt (22) herum angeordnet zu sein,
wobei, wenn sich der Verriegelungsring (6) um den Kopfaufnahmeabschnitt (22) herum befindet, der Verriegelungsring (6) eine Verriegelungsposition annehmen kann, in der der Verriegelungsring (6) eine Kraft auf den Kopfaufnahmeabschnitt (22) ausübt, um einen eingeführten Kopf (3) zu verriegeln; und
wobei der Durchgang (51) einen Aufweitungsabschnitt (26) umfasst, der sich von dem Sitzabschnitt (25) in Richtung des Bodens (12c) der Ausnehmung (12) aufweitet.

2. Kopplungsvorrichtung nach Anspruch 1, wobei der Aufweitungsabschnitt (26) eine konische Form aufweist.

3. Kopplungsvorrichtung nach Anspruch 1 oder 2, wobei der Durchgang (51) eine koaxiale Bohrung (11) in einem axialen Bereich umfasst, der sich entlang des Bodens (12c) der Ausnehmung (12) erstreckt, und wobei der Aufweitungsabschnitt (26) ein unteres Ende, das in Richtung des Sitzabschnitts (25) weist, und ein oberes Ende, das in Richtung des Bodens (12c) der Ausnehmung (12) weist, umfasst und wobei eine Innenbreite an dem oberen Ende des Aufweitungsabschnitts (26) größer als ein Durchmesser der Bohrung (11) ist.

4. Kopplungsvorrichtung nach Anspruch 3, wobei der Durchgang (51) zwischen dem oberen Ende des Aufweitungsabschnitts (26) und dem Boden (12c) der Ausnehmung (12) einen Übergangsabschnitt (27) umfasst, der sich in Richtung der Bohrung (11) verjüngt.

5. Kopplungsvorrichtung nach einem der Ansprüche 1 bis 4, wobei der Kopfaufnahmeabschnitt (22) benachbart zu dem zweiten Ende (5b) einen inneren konischen Oberflächenabschnitt (28) umfasst, der sich in Richtung des Sitzabschnitts (25) verengt und der eine axiale Länge (l) aufweist, die einem Abschnitt eines eingesetzten Kopfs (3) entspricht, der sich aus dem Sitzabschnitt (25) heraus erstreckt, wenn der Schaft (2) des Knochenverankerungselements koaxial zu der Mittelachse (C) ist.

6. Kopplungsvorrichtung nach Anspruch 5, wobei der innere konische Oberflächenabschnitt (28) einen Winkel mit der Mittelachse (C) von 30° oder mehr bildet.

7. Kopplungsvorrichtung nach einem der Ansprüche 1 bis 6, wobei der Kopfaufnahmeabschnitt benachbart zu dem zweiten Ende (5b) einen äußeren konischen Oberflächenabschnitt (29) umfasst, der sich in Richtung des zweiten Endes (5b) aufweitet, wobei vorzugsweise der äußere konische Oberflächenabschnitt einen Winkel mit der Mittelachse (C) bildet, der zwischen etwa 4° und 6°, weiter bevorzugt etwa 5° beträgt.

8. Kopplungsvorrichtung nach einem der Ansprüche 1 bis 7, wobei der Kopfaufnahmeabschnitt (22) eine Mehrzahl von sich axial erstreckenden Schlitzen (32a) eines ersten Typs umfasst, die in Richtung des zweiten Endes offen sind, und wobei mindestens einer der Schlitze (32a) ein geschlossenes Ende gegenüber dem zweiten Ende (5b) aufweist, das sich an einer axialen Position innerhalb des Aufweitungsabschnitts (26) befindet, wobei vorzugsweise mindestens einer, weiter bevorzugt zwei oder mehr der Schlitze (32a) des ersten Typs sich an einer Umfangsposition außerhalb der Position der Ausnehmung (12) befinden.

9. Kopplungsvorrichtung nach einem der Ansprüche 1 bis 8, wobei der Kopfaufnahmeabschnitt (22) eine Mehrzahl von sich axial erstreckenden Schlitzen (32b) eines zweiten Typs umfasst, die in Richtung des zweiten Endes (5b) offen sind, und wobei mindestens einer der Schlitze (32b) ein geschlossenes Ende gegenüber dem zweiten Ende (5b) aufweist, das sich an einer axialen Position näher an dem Sitzabschnitt (25) als an dem Boden (12c) der Ausnehmung (12) befindet, wobei vorzugsweise mindestens einer, weiter bevorzugt zwei oder mehr der Schlitze (32b) des zweiten Typs sich an einer Umfangsposition befinden, die der Position der Ausnehmung (12) entspricht.

10. Kopplungsvorrichtung nach Anspruch 8 oder 9, wobei die Schlitze (32a) des ersten Typs axial länger sind als die Schlitze (32b) des zweiten Typs.

11. Kopplungsvorrichtung nach einem der Ansprüche 1 bis 10, wobei die Schenkel (12a, 12b) verlängerte Laschen umfassen, die abgebrochen werden können.

12. Kopplungsvorrichtung nach einem der Ansprüche 1 bis 11, wobei der Durchgang (51) eine erste Bohrung (10) benachbart zu dem ersten Ende (5a) umfasst, wobei die erste Bohrung ein Innengewinde (13) umfasst und wobei die erste Bohrung (10) frei von einer Hinterschneidung an einem unteren Ende des Innengewindes (13) ist.

13. Kopplungsvorrichtung nach einem der Ansprüche 1 bis 12, wobei der Stabaufnahmeabschnitt (9) an einer Position unter dem Boden (12c) der Ausnehmung (12) Ausschnitte (20) an beiden Enden des Kanals zum Aufnehmen von Vorsprüngen (64) des Verriegelungsrings (6) darin umfasst und wobei eine Kontur der Ausschnitte (20) einer Kontur der Vorsprünge (64) entspricht, wobei die Kontur gerundet, vorzugsweise zylindrisch ist.

14. Kopplungsvorrichtung nach einem der Ansprüche 1 bis 13, wobei der Stabaufnahmeabschnitt (9) eine im Wesentlichen zylindrische Außenfläche mit einer ersten Außenbreite umfasst und wobei der Kopfaufnahmeabschnitt (22) benachbart zu dem Stabaufnahmeabschnitt (9) eine zweite Außenbreite umfasst, die kleiner als die erste Außenbreite ist, so dass eine Stufe zwischen einem unteren Ende (9b) des Stabaufnahmeabschnitts (9) und dem Kopfaufnahmeabschnitt (22) gebildet ist.

15. Kopplungsvorrichtung nach einem der Ansprüche 1 bis 14, wobei der Stabaufnahmeabschnitt (9) weiter eine Außenfläche und eine erste Eingriffsstruktur (15a, 15b) an der Außenfläche aufweist und der Verriegelungsring (6) eine Außenfläche und eine zweite Eingriffsstruktur (66, 66a) an der Außenfläche aufweist und wobei die erste Eingriffsstruktur (15a, 15b) und die zweite Eingriffsstruktur (66, 66a) dazu ausgebildet sind, mit einem Instrument in Eingriff zu sein, um den Verriegelungsring (6) relativ zu dem Aufnahmeteil (5) entlang der Mittelachse (C) von der Verriegelungsposition in Richtung des ersten Endes (5a) zu einer Position zu bewegen, in der der eingeführte Kopf (3) schwenkbar ist.

16. Polyaxiale Knochenverankerungsvorrichtung, die eine Kopplungsvorrichtung nach einem der Ansprüche 1 bis 15 und ein Knochenverankerungselement (1) mit einem Schaft (2) und einem Kopf (3) umfasst, wobei der Kopf (3) einen kugelförmigen Außenflächenabschnitt (3a) umfasst.

## Revendications

1. Dispositif de couplage pour coupler une tige (100) à un élément d'ancrage osseux (1) ayant une tête (3) et un manche (2),
le dispositif de couplage comprenant
une partie de réception (5) ayant une première extrémité (5a), une seconde extrémité (5b), un axe central (C) s'étendant entre la première extrémité (5a) et la seconde extrémité (5b) et un passage (51) s'étendant de la première extrémité (5a) à la seconde extrémité (5b) ; la partie de réception (5) comprenant en outre
une partie de réception de tige (9) ayant un évidement (12) définissant un canal pour recevoir la tige (100), l'évidement s'étendant de la première extrémité (5a) vers la seconde extrémité (5b) et formant deux branches libres (12a, 12b) et ayant un fond (12c) ; et
une partie de réception de tête (22) ouverte vers la seconde extrémité (5b) et comprenant une partie de siège (25) pour recevoir de manière pivotante la tête (3) de l'élément d'ancrage osseux (1), la partie de réception de tête étant flexible pour insérer et serrer la tête (3) ; et
une bague de verrouillage (6) configurée pour être agencée autour de la partie de réception de tête (22),
dans lequel, lorsque la bague de verrouillage (6) est autour de la partie de réception de tête (22), la bague de verrouillage (6) peut adopter une position de verrouillage dans laquelle la bague de verrouillage (6) exerce une force sur la partie de réception de tête (22) pour verrouiller une tête insérée (3) ; et
dans lequel le passage (51) comprend une section d'élargissement (26) qui s'élargit de la partie de siège (25) vers le fond (12c) de l'évidement (12).

2. Dispositif de couplage selon la revendication 1, dans lequel la section d'élargissement (26) a une forme conique.

3. Dispositif de couplage selon la revendication 1 ou 2, dans lequel le passage (51) comprend un alésage coaxial (11) dans une région axiale s'étendant le long du fond (12c) de l'évidement (12) et dans lequel la section d'élargissement (26) comprend une extrémité inférieure tournée vers la partie de siège (25) et une extrémité supérieure tournée vers le fond (12c) de l'évidement (12) et dans lequel une largeur interne au niveau de l'extrémité supérieure de la section d'élargissement (26) est supérieure à un diamètre de l'alésage (11).

4. Dispositif de couplage selon la revendication 3, dans lequel le passage (51) comprend entre l'extrémité supérieure de la section d'élargissement (26) et le fond (12c) de l'évidement (12) une section de transition (27) se rétrécissant vers l'alésage (11).

5. Dispositif de couplage selon l'une des revendications 1 à 4, dans lequel la partie de réception de tête (22) comprend adjacente à la seconde extrémité (5b) une partie de surface conique interne (28) qui se rétrécit vers la partie de siège (25) et qui a une longueur axiale (l) correspondant à une partie d'une tête insérée (3) qui s'étend hors de la partie de siège (25) lorsque le manche (2) de l'élément d'ancrage osseux est coaxial avec l'axe central (C).

6. Dispositif de couplage selon la revendication 5, dans lequel la partie de surface conique interne (28) forme un angle avec l'axe central (C) de 30° ou plus.

7. Dispositif de couplage selon l'une des revendications 1 à 6, dans lequel la partie de réception de tête comprend adjacente à la seconde extrémité (5b) une partie de surface conique externe (29) qui s'élargit vers la seconde extrémité (5b), de préférence dans lequel la partie de surface conique externe forme un angle avec l'axe central (C) qui est entre environ 4° et 6°, plus préférablement environ 5°.

8. Dispositif de couplage selon l'une des revendications 1 à 7, dans lequel la partie de réception de tête (22) comprend une pluralité de fentes s'étendant axialement (32a) d'un premier type qui sont ouvertes vers la seconde extrémité et dans lequel au moins l'une des fentes (32a) a une extrémité fermée opposée à la seconde extrémité (5b) qui est située à une position axiale à l'intérieur de la section d'élargissement (26), de préférence dans lequel au moins une, plus préférablement deux ou plus des fentes (32a) du premier type sont situées à une position circonférentielle à l'extérieur de la position de l'évidement (12).

9. Dispositif de couplage selon l'une des revendications 1 à 8, dans lequel la partie de réception de tête (22) comprend une pluralité de fentes s'étendant axialement (32b) d'un second type qui sont ouvertes vers la seconde extrémité (5b) et dans lequel au moins l'une des fentes (32b) a une extrémité fermée opposée à la seconde extrémité (5b) qui est située à une position axiale plus proche de la partie de siège (25) que du fond (12c) de l'évidement (12), de préférence dans lequel au moins une, plus préférablement deux ou plus des fentes (32b) du second type, sont situées à une position circonférentielle correspondant à la position de l'évidement (12).

10. Dispositif de couplage selon la revendication 8 ou 9, dans lequel les fentes (32a) du premier type sont axialement plus longues que les fentes (32b) du second type.

11. Dispositif de couplage selon l'une des revendications 1 à 10, dans lequel les branches (12a, 12b) comprennent des languettes étendues qui peuvent être rompues.

12. Dispositif de couplage selon l'une des revendications 1 à 11, dans lequel le passage (51) comprend un premier alésage (10) adjacent à la première extrémité (5a), le premier alésage comprenant un filetage interne (13) et dans lequel le premier alésage (10) est exempt d'une contre-dépouille au niveau d'une extrémité inférieure du filetage interne (13).

13. Dispositif de couplage selon l'une des revendications 1 à 12, dans lequel la partie de réception de tige (9) comprend à une position au-dessous du fond (12c) de l'évidement (12) des découpes (20) au niveau de chaque extrémité du canal pour recevoir des saillies (64) de la bague de verrouillage (6) et dans lequel un contour des découpes (20) correspond à un contour des saillies (64), dans lequel le contour est arrondi, de préférence cylindrique.

14. Dispositif de couplage selon l'une des revendications 1 à 13, dans lequel la partie de réception de tige (9) comprend une surface externe sensiblement cylindrique avec une première largeur externe et dans lequel la partie de réception de tête (22) comprend adjacente à la partie de réception de tige (9) une seconde largeur externe qui est inférieure à la première largeur externe de sorte qu'un gradin est formé entre une extrémité inférieure (9b) de la partie de réception de tige (9) et la partie de réception de tête (22).

15. Dispositif de couplage selon l'une des revendications 1 à 14, dans lequel la partie de réception de tige (9) a en outre une surface externe et une première structure de mise en prise (15a, 15b) sur la surface externe et la bague de verrouillage (6) a une surface externe et une seconde structure de mise en prise (66, 66a) sur la surface externe et dans lequel la première structure de mise en prise (15a, 15b) et la seconde structure de mise en prise (66, 66a) sont configurées pour être mises en prise par un instrument pour déplacer la bague de verrouillage (6) par rapport à la partie de réception (5) le long de l'axe central (C) de la position de verrouillage vers la première extrémité (5a) à une position dans laquelle la tête insérée (3) peut pivoter.

16. Dispositif d'ancrage osseux polyaxial comprenant un dispositif de couplage selon l'une quelconque des revendications 1 à 15 et un élément d'ancrage osseux (1) ayant un manche (2) et une tête (3) dans lequel la tête (3) comprend une partie de surface externe sphérique (3a).
